# EUROPEAN PATENT APPLICATION

(11) **EP 0 582 256 A2**
(43) Date of publication of application: **09.02.1994**
(21) Application number: 93112368.1
(22) Date of filing: 02.08.1993
(51) Int. Cl.: C12Q 1/68, B01D 57/02, G01N 27/447

(54) **Polynucleotide detecting method and apparatus**

(30) Priority: 06.08.1992 JP 210073/92
(71) Applicant: HITACHI, LTD., Chiyoda-ku, Tokyo 100 (JP)
(72) Inventor: Okano, Kazunori, Shiki-shi (JP); Kambara, Hideki, Hachioji-shi (JP); Nagai, Keiichi, Higashiyamato-shi (JP)
(74) Representative: Strehl Schübel-Hopf Groening & Partner

(57) **Abstract**

Fluorescent-labeled probes 2 hybridizable with target polynucleotide chain 1 are used as fluorescent-labeled probes, and both are reacted to obtain a fluorescent-labeled probe 3 bound to the target polynucleotide 1. After electrophoretically separating the unreacted excess fluorescent-labeled probes, electrophoresis is further continued at a temperature above the denaturing temperature of the complex 3 of target polynucleotide and fluorescent-labeled probe to release the fluorescent-labeled probe 4 which has been bound to the target polynucleotide. Laser beam 6 is applied to the released fluorescent-labeled probe 4 and the generated fluorescence is detected by fluorescence detector 7. Thereby, the present invention enables successive automatic prosecution of a series of operating step and provides a high-sensitivity and high-precision quantitative polynucleotide detecting method suited for application to a high-throughput automated detecting apparatus.

## Description

### BACKGROUND OF THE INVENTION

### Field of Invention

The present invention relates to a method for detecting polynucleotides such as DNA and RNA and an apparatus therefor.

### Description of the Related Art

A conventional polynucleotide detecting method comprising associating a labeled polynucleotide bound to a labeling material with a target polynucleotide (DNA or RNA) sample is described in Proc. Natl. Acad. Sci. USA 80, 278-282 (1983). According to this method, a fragmental specimen of an electrophoretically separated polynucleotide is transferred to a nitrocelluose membrane and fixed, and then this membrane is immersed in a solution containing the radioisotope-labeled polynucleotide probes and subjected to a hybridization reaction. After the reaction, the unreacted labeled probes are washed away from the membrane, and then the amount of the residual labeled probes bound to the membrane is detected by auto-radiography to thereby determine the presence or absence of the target polynucleotide.

Also, a method for detecting a strange target polynucleotide such as a bacterium or virus in a specimen such as blood or excrement is disclosed in JP-A-58-31998. According to this method, the polynucleotide in a refined specimen is denatured into single strand by heating or other means and fixed to a nitrocellulose membrane. Then, the polynucleotide of the bacterium or virus to be tested is reacted with the radioisotope-labeled polynucleotide probe having a complementary base sequence, and thereafter, the membrane is washed. If the polynucleotide of the particular bacterium or virus is contained in the specimen, the labeled polynucleotide probe is associated therewith as a result of hybridization reaction and left on the membrane. This aggregate is detected by auto-radiography to determine the presence or absence of the target polynucleotide.

### SUMMARY OF THE INVENTION

In the conventional methods described above, a radioisotope is required for labeling of the polynucleotide probes. These methods take a long time for detection and often prove too low in sensitivity. This is attributable to low relative radio-activity per probe as it is impossible to label with more than a tracer amount of radioisotope and background radiation associated with non-specific binding of the probes to the nitrocellulose membrane. Also, the conventional methods are incapable of quantitative determination; they can only determine the presence or absence of the target polynucleotide or make merely semi-quantitative determination from photographic density of the auto-radiographic film. Further, the conventional methods have difficulties for automation because of use of auto-radiography, and it is impossible to examine a large number of specimens in a clinical test since the operator is subject to radiation exposure.

Recently, enzymes and chemical luminous substances have come to be used as labeling material for improving the quantification performance or preventing radiation exposure. Nevertheless, there still remain unsolved the problems of inaptitude of the handling of the nitrocellulose and other membranes for automation and a wide scatter of determinations and insufficient detecting sensitivity due to non-specific binding of the specimen or labeling material to the membrane. Therefore, where a high sensitivity is required, a method is generally employed in which the polynucleotide to be determined is previously proliferated by using polymerase (PCR (polymerase chain reaction)method; Nature 335, 414-417, 1988). In the PCR method, the following sequential operations ① - ③ are repeated cyclically: ① primers are bound to the separated positions in each of the two chains of the target polynucleotide; ② a complementary chain is synthesized with each primer acting as starting point by using polymerase; and ③ the synthesized complementary chain is dissociated by heating. According to this method, since the once synthesized complementary chain becomes the template polynucleotide in the next cycle of the above operations, the complementary chain synthesized upon every cycle of said operations increases in geometrical progression. Repetition of several ten times of said cycle of operations proliferates the initial target polynucleotide about 100,000-fold. In the PCR method, however, since polymerase is apt to become active when the primer and the polynucleotide are not perfectly complementary to each other, a problem is posed that the polynucleotides other than the target polynucleotide might be proliferated. Also, the PCR method is subject to disturbance by the contaminants other than the target polynucleotide due to high proliferation rate. Further in the PCR method, since the polynucleotide increases by geometrical progression, it is unable to give a wide dynamic range for determination when using an ordinary determining method, and also there is the problem on precision of determination, so that it is hardly possible to make a correct quantitative determination.

Accordingly, the primary object of the present invention is to provide a high-sensitivity and high-precision quantitative polynucleotide detecting method. Another object of the present invention is to realize use of a polynucleotide probe with no radioisotope and to provide a polynucleotide detecting method suited for application to a high-throughput automated detecting apparatus.

In the present invention, in order to realize the use of a polynucleotide probe with no radioisotope, a fluorescent substance is used as labeling material.

Also, in order to realize high-sensitivity quantitative determination of polynucleotide, the following techniques are employed in the present invention.
(1) After the reaction of fluorescent-labeled probes and target polynucleotide under the hybridization condition, the unreacted fluorescent-labeled probes are electrophoretically separated (removed) from a complex of target polynucleotide and fluorescent-labeled probes, then the complex is denatured, and the fluorescent-labeled probe bound to the target polynucleotide is electrophoretically separated and determined. Usually the target polynucleotide has a greater molecular size than the fluorescent-labeled probes, so that the latter are higher in electrophoretic mobility. It is therefore easy to separate the unreacted fluorescent-labeled probes by difference in the mobility from the complex of target polynucleotide and fluorescent-labeled probes. Especially in case the target polynucleotide has several thousands of or more bases, there takes place almost no electrophoretic migration of molecules under a non-denaturing condition when carrying out the denaturing and hybridization treatments, so that electrophoretic removal of the excess amount of fluorescent-labeled probes is effective. After removal of the unreacted fluorescent-labeled probes, the complex of target polynucleotide and fluorescent-labeled probes is denatured and further subjected to electrophoresis, which causes elution of the fluorescent-labeled probe bound to the target polynucleotide. Since the eluted fluorescent-labeled probe contains no unreacted fluorescent-labeled probe, the quantity of the target polynucleotide can be known accurately by determining the intensity of fluorescence radiating from the eluted fluorescent-labeled probe.
(2) Denaturing of the complex of target polynucleotide and fluorescent-labeled probes can be accomplished by means of heating or addition of a denaturing agent such as urea, formamide or the like, or by combination of said both means. In the case of heating, there is usually employed a method in which the electrophoretic matrix is heated by hot water or by a heater. Other methods such as heating the electrophoretic matrix with Joule heat by elevating the electrophoretic voltage or by utilizing the microwaves or infrared rays are usable, but it is preferred to use the method of direct heating of the electrophoretic matrix by a Peltier device or the method utilizing Joule heat by controlling the electrophoretic voltage, or combination of both methods, as these methods allow rapid rise of temperature and control thereof. A method using a denaturing agent is also effective since the complex of target polynucleotide and fluorescent-labeled probes makes almost no migration and stays near the sample injection member. When a denaturing agent is added to the sample injection member, the denaturing agent is diffused to reach the complex to denature it. As the denaturing agent, urea or formamide is preferred. However, in the case of a complex of target polynucleotide and fluorescent-labeled probes with high GC content, it is especially effective to apply heating with addition of a denaturing agent.
(3) By carrying out electrophoresis while raising the temperature continuously or stepwise by making use of a Peltier device or Joule heat, it is possible to remove the fluorescent-labeled probes bound non-specifically to the polynucleotides other than the target polynucleotide which have got mixed in the sample.
(4) In the present invention, in order to provide a polynucleotide detecting method suited for application to a high-throughput automatic detector, the electrophoretic operation is divided into two steps for carrying out electrophoresis by using the different electrophoretic matrices in the respective steps. That is, after removing the unreacted fluorescent-labeled probes from the complex of target polynucleotide and fluorescent-labeled probes, the electrophoretic matrix containing the complex is contacted with another electrophoretic matrix from which a fluorescent-labeled probe is to be separated and determined, and then this fluorescent-labeled probe is eluted from the target polynucleotide under a denaturing condition and determined. According to this process, it is possible to keep a denaturing agent present in the latter electrophoretic matrix or to leave the matrix always in a heated state.
(5) The matrix used in the step of separating and removing the unreacted fluorescent-labeled proves from the complex of target polynucleotide and fluorescent-labeled probes is made of polyacrylamide, cellulose, fluorocarbon or other like substance. The matrix is a gel or a film having pores which do not allow passage of the target polynucleotide particles. The smaller the film thickness, the more favorable for the next denaturing operation, as far as the film is capable of capturing the complex of target polynucleotide and fluorescent-labeled probes. It is desired for fluorescent determination that the electrophoretic matrix used in the step of separating a fluorescent-labeled probe from the complex and determining the separated probe is principally composed of polyacrylamide, or that no matrix is used at all. For determining the fluorescent-labeled probe, there are used an exciting light source for illuminating the fluorescence-determined portion of the electrophoretic matrix, a multiplier phototube or a two-dimensional photon counter or a light receptor such as CCD array for determining fluorescence, and a detector comprising a device for processing the signals from the light receptor. Here, laser light is preferably used as light source because the exciting light can be condensed strongly.

The present invention may be summarized as follows. First, a sample and the fluorescent-labeled probes are reacted to form a complex thereof, and this complex is subjected to electrophoresis to remove the unreacted fluorescent-labeled probes. The complex scarcely undergoes migration at this point. Then the complex is denatured by addition of a denaturing agent or by heating. As electrophoresis is further continued, the fluorescent-labeled probe which has been bound to the target polynucleotide is released and begins to migrate, and this probe is detected.

According to the present invention, since the unreacted fluorescent-labeled probes and non-specifically reacted fluorescent-labeled probes can be separated from the fluorescent-labeled probe specifically hybridized with the target polynucleotide, it is possible to detect the target polynucleotide with high sensitivity. Also, the present invention enables successive automatic prosecution of a series of operating steps comprising hybridization reaction, separation of unreacted fluorescent-labeled probes and elution and detection of the specifically hybridized fluorescent-labeled probe. Further, the present invention incorporates a random-access system which allows selection of a desired sample and a desired fluorescent-labeled probe by simple turn of a turn stage, so that the present invention can be effectively applied to the field of clinical testing.

### BRIEF DESCRIPTION OF THE DRAWINGS

Fig. 1 is a conceptual illustration of the polynucleotide detection method according to the present invention.

Fig. 2 is a schematic illustration of the reaction apparatus according to the present invention.

Fig. 3 is a schematic illustration of the electrophoretic apparatus and the fluorescence detecting section according to the present invention.

Fig. 4 is a graphic representation of the result of a detection test on a sample according to the present invention.

Fig. 5 shows a calibration curve of the target polynucleotide obtained according to the process of the present invention.

Fig. 6 is a schematic illustration of the electrophoretic apparatus and the fluorescence detecting section according to the present invention.

### PREFERRED EMBODIMENTS OF THE INVENTION

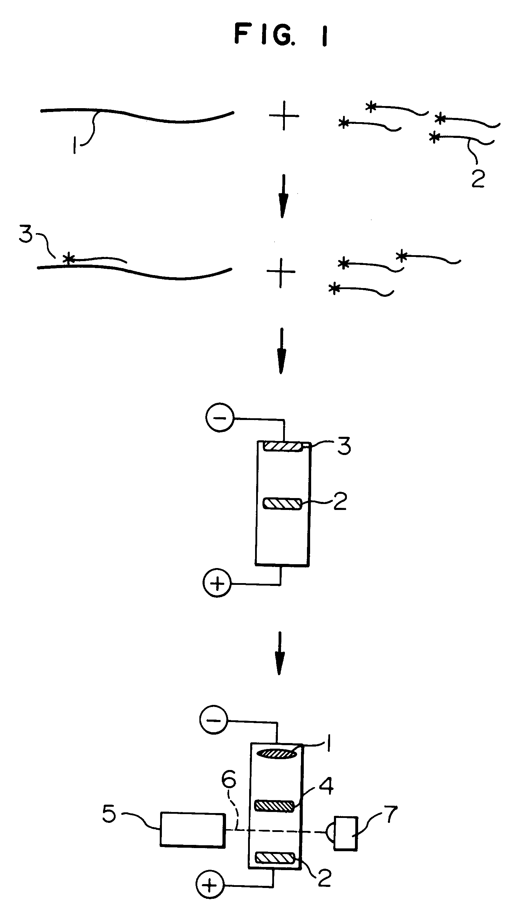

The concept of the present invention is explained with reference to Fig. 1. There are used the fluorescent-labeled probes 2 hybridized with the target polynucleotide chain 1. When they are reacted with each other, a fluorescent-labeled probe 3 bound to the target polynucleotide is obtained. The fluorescent-labeled probe 3 bound to the target polynucleotide and the unreacted excess fluorescent-labeled probes can be easily separated electrophoretically because of the difference in molecular size. As the fluorescent substance, there can be used the family of fluorecein, the family of rhodamine, 4-nitrobenzo-2-oxa-1,3-diazole, phthalocyanine, etc., and derivatives thereof, but it is preferred to use sulforhodamine 101 which is a long wavelength fluorophore having a high molecular extinction coefficient and high quantum yield. After removing the excess fluorescent-labeled probes, electrophoresis is further continued at a temperature above the denaturing temperature of the complex 3 of target polynucleotide and fluorescent-labeled probes, whereby a fluorescent-labeled probe 4 bound to the target polynucleotide is released. When the released fluorescent-labeled probe 4 intersects the laser beam 6 transmitted from a laser generator 5, fluorescence originating in the fluorescent-labeled probe is emitted. This fluorescence can be detected by a fluorescence detector 7. Since the detected quantity of fluorescence is in a quantitative relation with the target polynucleotide chain 1, it is possible to detect and quantify the target polynucleotide in the sample.

The length of the fluorescent-labeled probe is not defined as far as said probe can be bound specifically to the target polynucleotide chain, but it is preferably in a range from 12 to several ten bases in length. If the probe length is shorter than this range, the reactions non-specific to the polynucleotides other than the target polynucleotide increase. Chemical synthesis is recommended for obtaining the fluorescent-labeled probes of various sequences. The fluorescent-labeled probes of any length not exceeding 100 bases in length can be easily synthesized. For binding the fluorescent substance to the synthesized polynucleotide probe, an amino group is bound to the polynucleotide probe at the final stage of synthesis thereof, and a fluorescent pigment having a functional group, for example, sulforhodamine 101 acid chloride, is reacted with the amino group.

### Example 1

In this example, an M13 mp8 single-stranded DNA was used as target polynucleotide sample.

The probe complemental to this target DNA is a single-stranded polynucleotide bound complimentarily to #6269-6292 of the base sequence of the target M13 mp8 single-stranded DNA. This single-stranded polynucleotide possesses an amino group at its 5' terminal and also has the following base sequence:
CGACGTTGTAAAACGACGGCCAGT
This probe has sulforhodamine 101 bound through an amino group to its 5' terminal where the probe is bound complimentarily to the target polynucleotide. A process for preparing this fluorescent-labeled probe is described below.

At the final stage of synthesis of the synthetic polynucleotide, N-monomethoxytritylaminohexa-6-oxy-β-cyanoethyl-N, N-diisopropylaminosulfoamidite is reacted with the polynucleotide by a known method to introduce an amino group to the 5' terminal, followed by a further reaction with 100 times the molar quantity of sulforhodamine 101 acid chloride in a 0.1 M carbonate buffer (pH 9). The resulting fluorescent-labeled probe is refined by ethanol precipitation method and polyacrylamide gel electrophoresis. The thus obtained fluorescent-labeled probe is an electrophoretically pure product.

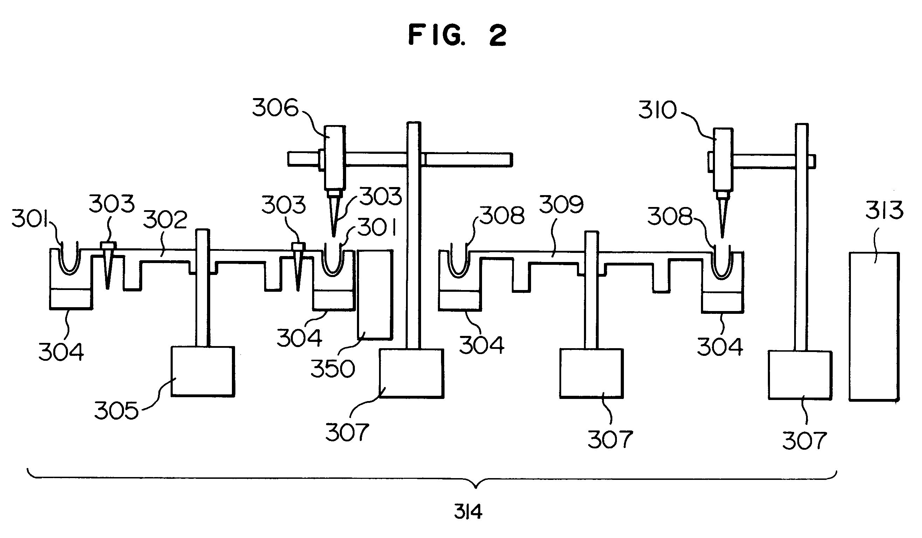

The hybridization reaction is carried out according to the following procedure by using a reaction apparatus 314 such as shown in Fig. 2. The target M13 mp8 single-stranded DNA solutions of various concentrations are placed in the vessels 301 and these vessels are set on a vessel holding stage 302. The labeled proves are also placed in the similar vessels set on the vessel holding stage 302. On the vessel holding stage, there are also provided the disposable pipette tips 303 to be used for the respective reaction vessels. The sample in each vessel 301, if necessary, may be heated by a heater 304 to denature the sample. The vessel holding stage 302 is a disc which can be turned by the operation of a stepping motor 305 so that a desired vessel may be set at the position of an auto-pipette 306. This auto-pipette 306 is designed to be able to turn horizontally and move up and down by the operation of a stepping motor 307 so that said pipette can take a desired position between the vessel holding stage 302 and the reaction stage 309.

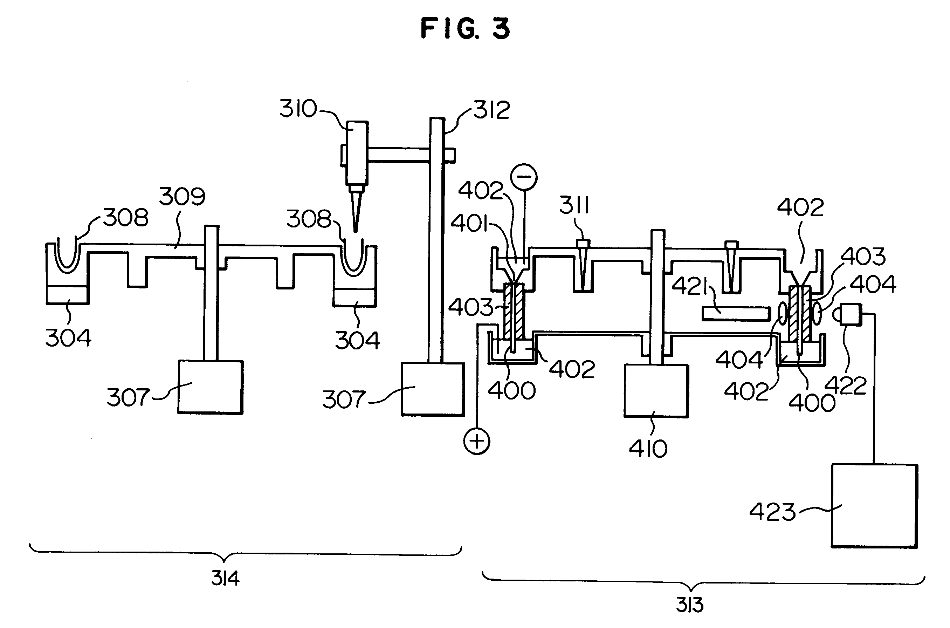

First of all, the auto-pipette 306 is moved to a proper position and a pipette tip 303 is attached thereto. Then the sample in a vessel 301 is sucked up in an amount of 25 µl by the pipette tip and dropped into a reaction vessel 308 brought to the pipetting position on the reaction stage 309. The used pipette tip is thrown away into a tip-dumper 350. Similarly, 25 µl of labeled probe is sucked up from a different vessel on the vessel holding stage 302 and pipetted into the reaction vessel 308 on the reaction stage 309. The reaction stage 309 is designed to be turned by the operation of a stepping motor 307 so that a plurality of reaction vessels may be successively brought to the pipetting position where a sample solution and labeled probe are pipetted into the vessel placed there. Also, each of the reaction vessels 308 on the reaction stage 309 is provided with a heater 304 whereby the reactants in each reaction vessel can be heated to and kept at a given temperature independently of the other vessels. Here, the reactants are heated to and kept at 95°C for one minute and then reacted at 42°C for 30 minutes. The reaction apparatus 314 of Fig. 2 is associated with an electrophoretic region 313. After the reaction, the reaction solution is pipetted into a sample injection member 401 of an electrophoretic matrix 400 by the auto-pipette 310 as shown in Fig. 3. The electrophoretic matrix 400 is set on an electrophoretic matrix holding stage which is designed to be turned by the operation of a stepping motor 410. First, a pipette tip 311 is attached to the auto-pipette 310 by using a lift 312. The pipette tips are provided on the electrophoretic matrix holding stage. The electrophoretic matrices are successively supplied to the pipetting position as the electrophoretic matrix holding stage is turned. Each electrophoretic matrix is 2 cm long and its sample injection member is cone-shaped and has a volume of 100 µl. Each electrophoretic matrix 400 is furnished with an electrode cell 402 and a temperature controller 403. First, electrophoresis is carried out at 42°C and 10 v/cm to remove the unreacted labeled probes. The target M13 mp8 single-stranded DNA bound to a labeled probe scarcely makes electrophoretic migration and is therefore concentrated at the inlet of the reactants. Then the temperature is raised to 55°C and electrophoresis is further continued to remove the fluorescent-labeled probes bound non-specifically to the polynucleotides other than the target polynucleotide. Finally, the electrophoretic matrix is moved to the position of a fluorescence detecting unit and electrophoresis is carried on at 80 v/cm with the temperature set at 95°C by the temperature controller 403. The fluorescence detecting unit consists of a laser generator 421, a fluorescence detector 422 and a data processor 423. The fluorescence generated when the fluorescent-labeled probe released from the target M13 mp8 polynucleotide intersects the laser beam is detected by the fluorescence detector. The laser used here is 594 nm He/Ne laser, which is condensed by a condenser lens 404 in a detector multiplier phototube.

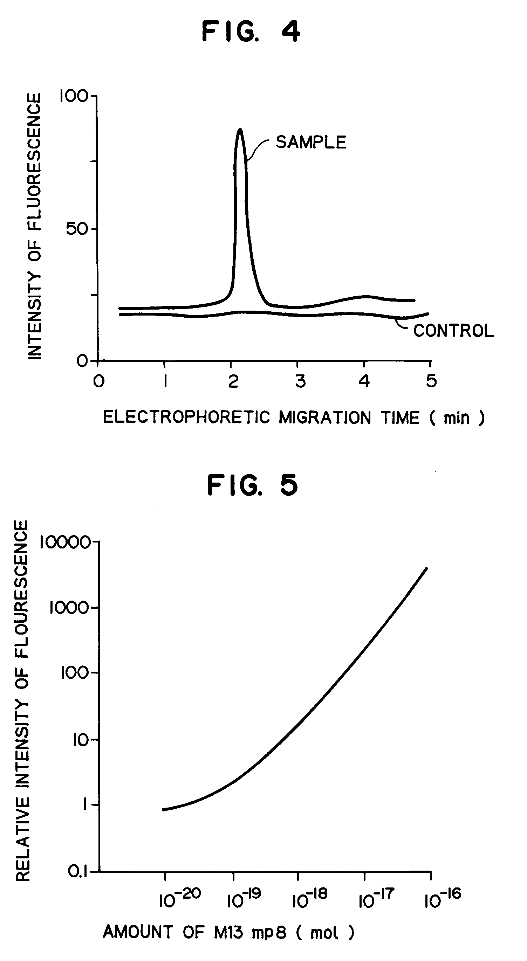

Fig. 4 shows the change of fluorescence with time during electrophoresis when a target M13 mp8 single-stranded DNA was used as target polynucleotide sample. The fluorescent-labeled probe which has been bound to the target M13 mp8 single-stranded DNA can be clearly detected. Fig. 5 shows a calibrication curve of fluorescence as determined by using the target M13 mp8 single-stranded DNA's of different concentrations as samples.

The present invention has the advantage that since the unreacted fluorescent-labeled probes or non-specifically reacted fluorescent-labeled probes can be separated from the fluorescent-labeled probe non-specifically hybridized with the target polynucleotide, it is possible to detect the target polynucleotide with high sensitivity. It is also a noticeable advantage of the present invention that it allows successive automatic prosecution of a series of operating steps comprising hybridization reaction, separation of unreacted fluorescent-labeled probes and elution and detection of the specifically hybridized fluorescent-labeled probe. Further, the present invention has realized a random-access system which allows selection of a desired sample and a desired fluorescent-labeled probe by simple turn of a turn stage. The present invention, therefore, can be effectively applied to the field of clinical testing.

In this example, the temperature of the electrophoretic matrix is raised stepwise, but a similar effect can be obtained when the temperature is raised continuously from 42°C to 95°C. It should be noted, however, that a too high temperature raising rate causes insufficient separation of the non-specifically bound fluorescent-labeled probes from the fluorescent-labeled probe bound to the target M13 mp8 single-stranded DNA, so that the temperature raising rate is preferably set at 5°C/min or below when the electrophoresis voltage is 10 v/cm.

### Example 2

This example concerns the case where there are used two different electrophoretic matrices, one for separation of the unreacted fluorescent-labeled probes and the other for determination of the fluorescent-labeled probe bound to the target polynucleotide. The samples and the fluorescent-labeled probes are the same as used in Example 1. When separation of the unreacted fluorescent-labeled probes and determination of the fluorescent-labeled probe bound to the target polynucleotide are performed by using a same electrophoretic matrix as in Example 1, a long time is required for the electrophoresis as it is necessary to separate a large excess of unreacted fluorescent-labeled probes and a small quantity of fluorescent-labeled probe bound to the target polynucleotide. Approximately 40 minutes are required for removing the unreacted fluorescent-labeled probes when the electrophoresis is carried out using a 2 cm electrophoretic matrix at an electrophoresis voltage of 10 v/cm as in Example 1. On the other hand, when there is provided a thin electrophoretic matrix to be used exclusively for separation of the unreacted fluorescent-labeled probes as in the instant example, it is possible to quickly remove the unreacted fluorescent-labeled probes since the unreacted fluorescent-labeled probes are not required to migrate in the electrophoretic matrix for determination of fluorescence.

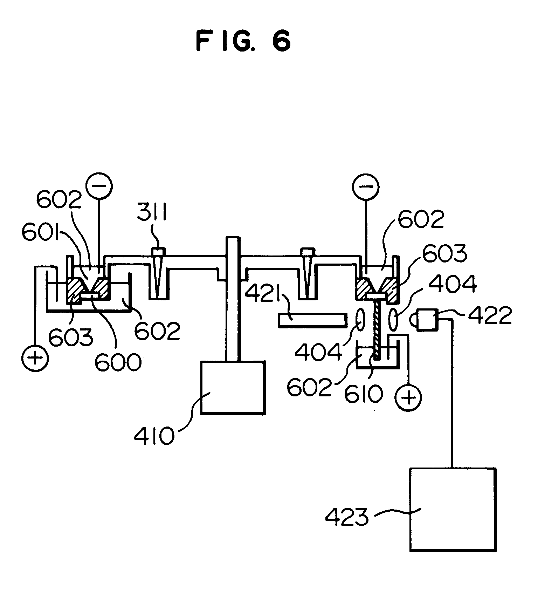

The electrophoretic matrix for separation of the unreacted fluorescent-labeled probes is made of a cellulose acetate membrane having a diameter of about 0.5 mm and a molecular weight cutoff of 10,000 daltons. To this membrane 600, as shown in Fig. 6, is connected a conical sample injection member 601 as in Example 1. The volume of the sample injection member is 100 µl. The electrophoretic matrix for separation of the unreacted fluorescent-labeled probes has connected to an electrode cell 602 and a temperature controller 603. Fifty microliters of a sample prepared in Example 1 is given to the sample injection member 601. The specific gravity of the sample is elevated with 20% sucrose or 10% glycerin. The sample is subjected to electrophoresis in 10 minutes at 42°C and 10 v/cm to remove the unreacted fluorescent-labeled probes, followed by additional electrophoresis at 55°C for 10 minutes to get rid of the fluorescent-labeled probes bound non-specifically to other polynulceotides than the target M13 mp8 single-stranded DNA. The electrophoretic matrix for separation of probes is laid on a turn table which can turn horizontally and move up and down by the operation of a stepping motor 410. Then the electrophoretic matrix for separation of the unreacted fluorescent-labeled probes is moved and attached to a 2 cm long electrophoretic matrix 610 for detection of fluorescence. This electrophoretic matrix for detection of fluorescence is made of polyacrylamide of 6% concentration, and it is packed in a quartz capillary tube and connected to an He/Ne laser and a multiplier phototube for detection of fluorescence. When electrophoresis was carried out at 80 v/cm by setting the temperature at 95°C by the temperature controller 604, a peak of fluorescence attributable to the fluorescent-labeled probe released from the target M13 mp8 single-stranded DNA is detected in about 2 to 4 minutes.

When a very thin electrophoretic matrix for exclusive use for separation of the unreacted fluorescent-labeled probes is provided as in this example, since the unreacted fluorescent-labeled probes are not required to migrate in the electrophoretic matrix for determination of fluorescence, it is possible to quickly remove the unreacted fluorescent-labeled probes, making it possible to shorten the electrophoresis time. Also, since removal of the unreacted fluorescent-labeled probes and the non-specifically reacted fluorescent-labeled probes becomes easy, disturbance by background fluorescence originating in these fluorescent-labeled probes is lessened, allowing high-accuracy determination of fluorescence.

## Claims

1. A polynucleotide detecting method comprising the steps of:
(i) binding a fluorescent-labeled probe to a target polynucleotide under a hybridization condition to obtain a complex of said target polynucleotide and said fluorescent-labeled probe;
(ii) electrophoretically separating away unreacted fluorescent-labeled probes from a mixture of said complex and unreacted fluorescent-labeled probes;
(iii) denaturing said complex and electrophoretically separating the fluorescent-labeled probe which has been bound to said target polynucleotide; and
(iv) exciting the fluorophore of said fluorescent-labeled probe to generate fluorescence and determining such fluorescence.

2. A method according to Claim 1, wherein a denaturing agent is added to the electrophoretic matrix used for the electrophoresis in step (iii) to denature said complex.

3. A method according to Claim 2, wherein the denaturing agent is urea or formamide.

4. A method according to Claim 1, wherein electrophoresis is carried out by denaturing said complex while raising, either continuously or stepwise, the temperature of the electrophoretic matrix used in the electrophoresis in step (iii).

5. A method according to Claim 1, wherein a denaturing agent is added to the electrophoretic matrix used in the electrophoresis in step (iii) and electrophoresis is carried out by denaturing said complex while raising, either continuously or stepwise, the temperature of said electrophoretic matrix.

6. A method according to Claim 1, wherein the electrophoretic matrix used for the electrophoresis in step (ii) and the electrophoretic matrix used for the electrophoresis in step (iii) are separate electrophoretic matrices.

7. A method according to Claim 1, wherein the electrophoretic matrix used for the electrophoresis in step (iii) is a gel or a membrane having pores not passing said target polynucleotide and made of a material selected from the group consisting of polyacrylamide, cellulose and fluorocarbon, and the electrophoretic matrix used for the electrophoresis in step (iii) is mainly composed of polyacrylamide, or no such matrix is used.

8. A polynucleotide detecting apparatus comprising:
a first electrophoretic means for separating away unreacted fluorescent-labeled probes from a mixture of a complex of a target polynucleotide and fluorescent-labeled probe and the unreacted fluorescent-labeled probes, said complex having been obtained by binding the fluorescent-labeled probe to the target polynucleotide under a hybridization condition;
a second electrophoretic means for denaturing said complex and separating the fluorescent-labeled probe bound to said target polynucleotide;
a means for exciting the fluorophore of said fluorescent-labeled probe: and
a means for detecting the fluorescence generated by said exciting means.

9. An apparatus according to Claim 8, wherein a denaturing agent is added to the electrophoretic matrix constituting said second electrophoretic means to denature said complex.

10. An apparatus according to Claim 9, wherein the denaturing agent is urea or formamide.

11. An apparatus according to Claim 8, wherein electrophoresis is carried out by denaturing said complex while raising, either continuously or stepwise, the temperature of the electrophoretic matrix constituting said second electrophoretic means.

12. An apparatus according to Claim 8, wherein a denaturing agent is added to the electrophoretic matrix constituting said second electrophoretic means and electrophoresis is carried out by denaturing said complex while raising, either continuously or stepwise, the temperature of said electrophoretic matrix.

13. An apparatus according to Claim 8, wherein the electrophoretic matrix constituting said first electrophoretic means and the electrophoretic matrix constituting said second electrophoretic means are separate electrophoretic matrices.

14. An apparatus according to Claim 8, wherein the electrophoretic matrix constituting said first electrophoretic means is a gel or a membrane having pores not passing said target polynucleotide and made of a material selected from the group consisting of polyacrylamide, cellulose and fluorocarbon, and the electrophoretic matrix constituting said second electrophoretic means is mainly composed of polyacrylamide, or no such matrix is used.
